**Europäisches Patentamt**

**European Patent Office**

**Office européen des brevets**

(19)

(11) Veröffentlichungsnummer : **0 097 366**
**B1**

(12) # EUROPÄISCHE PATENTSCHRIFT

(45) Veröffentlichungstag der Patentschrift :
07.09.88

(51) Int. Cl.⁴ : **A 61 M   1/16, B 01 D 13/00**

(21) Anmeldenummer : 83106063.7

(22) Anmeldetag : 21.06.83

(54) Dialysevorrichtung mit geregelter Zusammensetzung der Dialysierlösung.

(30) Priorität : 21.06.82 DE 3223051
15.06.83 DE 8317394 U

(43) Veröffentlichungstag der Anmeldung :
04.01.84 Patentblatt 84/01

(45) Bekanntmachung des Hinweises auf die Patenterteilung : 07.09.88 Patentblatt 88/36

(84) Benannte Vertragsstaaten :
AT BE CH DE FR GB IT LI LU NL SE

(56) Entgegenhaltungen :
EP-A- 0 029 793
DE-A- 2 419 516
DE-A- 2 644 584
DE-A- 2 745 572
DE-A- 2 838 414
DE-B- 1 766 008

(73) Patentinhaber : Fresenius AG
Gluckensteinweg 5
D-6380 Bad Homburg v.d.H. (DE)

(72) Erfinder : Polaschegg, Hans-Dieter, Dr.
Grünwiesenweg 9
D-6370 Oberursel 4 (DE)
Erfinder : Husar, Dieter, Dr.
Holzhäuserstrasse 6
D-6380 Bad Homburg (DE)

(74) Vertreter : Luderschmidt, Wolfgang, Dr. Dipl-Chem.
Görtz, Dr. Fuchs, Dr. Luderschmidt Patentanwälte
Sonnenberger Strasse 100 Postfach 26 26
D-6200 Wiesbaden (DE)

EP 0 097 366 B1

## Beschreibung

Die Erfindung betrifft eine Vorrichtung zum Reinigen von Blut gemäß dem ersten Teil der Ansprüche 1, 2 und 5.

Dialysevorrichtungen der eingangs erwähnten Art sind bekannt. Sie weisen als Detektor, der stromauf des Dialysators angeordnet ist, üblicherweise eine Leitfähigkeitsmeßzelle auf, mit der der temperaturkompensierte Leitfähigkeitswert der Dialysierlösung gemessen werden kann. Dieser Leitfähigkeitswert gibt die exakte Elektrolytzusammensetzung der Dialysierlösung wieder, so daß durch eine Änderung dieses Werts auf eine Änderung des Elektrolytgehalts der Dialysierlösung geschlossen werden kann.

Der Detektor selbst dient sowohl zur Einstellung der Elektrolytgehalts der Dialysierlösung als auch zur Abschaltung der gesamten Vorrichtung, sofern ein für den Patienten kritischer Zustand hierdurch erzeugt werden könnte.

Zur Regelung der Elektrolytzusammensetzung der Dialysierlösung steuert die als Detektor eingesetzte Leitfähigkeitsmeßzelle eine Pumpe, die das Konzentrat aus einem Konzentratvorratsbehälter in die Mischeinrichtung pumpt. Die Mischeinrichtung ist andererseits mit einem Leitungswasseranschluß versehen, über den gesteuert Leitungswasser zugeführt wird. In der Mischeinrichtung selbst erfolgt die gesteuerte Vermischung und Erwärmung von Leitungswasser und Konzentrat, wobei am Ausgang dieser Einrichtung die gewünschte Zusammensetzung der Dialysierlösung erhalten wird.

Diese Dialysierlösung wird durch den Dialysator geleitet, in dem die reinigung des Blutes von harnträchtigen Substanzen und der Entzug von Flüssigkeit erfolgen.

Infolge der hohen Austauschleistung (Clearance) der heute eingesetzten Dialysatoren werden harnträchtige Substanzen sehr rasch aus dem Blut entfernt, und es wird dadurch die Dialysezeit verringert. So kann bei hocheffektiven Dialysatoren die Behandlungszeit auf 3 × 2 Stunden wöchentlich verkürzt werden, innerhalb der nicht nur die harnträchtigen Substanzen, beispielsweise Harnstoff, sondern auch der Flüssigkeitsüberschuß entfernt werden.

Die Entfernung des Flüssigkeitsüberschusses erfordert eine sehr präzise Steuerung der Flüssigkeitsbilanzierung, weshalb dieses Verfahren auch nur mit flüssigkeitsbilanzierenden Vorrichtungen durchgeführt werden kann. Trotz dieser präzisen Bilanzierung kommt es immer noch zu dialysetypischen Unannehmlichkeiten bei den Patienten, so zu Kopfweh, Brechen und Muskelkrämpfen, was als « Disäquilibriumssyndrom » bezeichnet wird. Der Grund hierfür liegt vermutlich im zu raschen Entzug von Natriumionen aus dem Blut, der aufgrund der Konzentrationsdifferenz vom Natrium im Blut (extracorporaler Kreislauf) und in der Dialysierflüssigkeit erfolgt. Je größer die Austauschleistung des Dialysators ist, desto geringer darf der zulässige Gradient der Natriumkonzentration zwischen Blut und Dialysierflüssigkeit sein. So sollte eine Differenz von maximal 10 mmol/l Natrium bei normalen Dialysatoren zulässig sein, die sich bei Hochleistungsdialysatoren auf die Hälfte reduziert.

Nun differiert aber der Natriumgehalt im Blut der Patienten und liegt üblicherweise noch außerhalb des Normalbereichs von 135-147 mmol/l. Um die vorstehend beschriebenen Dialysesymptome zu vermeiden, arbeitet man vorteilhaft mit einer Natriumkonzentration in der Dialysierflüssigkeit von etwa 144 mmol/l. Die Folge davon ist, daß der Patient bei der Behandlung Durst bekommt und bis zur nächsten Dialysebehandlung relativ viel Flüssigkeit aufnimmt, also überwässert wird. Nicht selten wurden dabei Übergewichte bis zu 6 kg beobachtet. Diese zugenommene Flüssigkeitsmenge muß dann innerhalb der Behandlungsdauer von etwa 2-3 Stunden ultrafiltriert werden, wobei zusammen mit dieser Flüssigkeitsmenge die erforderliche Menge Natrium entzogen wird. Allerdings ist diese Behandlungsmethode nicht so exakt, daß die vorstehenden Symptome dadurch vermieden werden könnten.

Weiterhin ist die zwischen den Behandlungen auftretende starke Überwässerung des Organismus keineswegs zuträglich, kann jedoch andererseits bei den heute eingesetzten Verfahren nicht vermieden werden.

Es wurden daher bereits Geräte (beispielsweise Seratron der Firma Cordis-Dow) entwickelt, die ausgehend von einer Dialysierlösung mit bestimmter Zusammensetzung über die Dialysezeit diese Zusammensetzung verändert, wobei diese Veränderung nach einem bestimmten Programm erfolgt. Dieses Verfahren ist als « Natriummodelling » bekannt. Dies Programm weist natürlich den Nachteil auf, daß es keineswegs auf die individuellen Gegebenheiten zugeschnitten ist. Infolge der fest eingestellten Anfangskonzentrationen kommt es bereits zu Schwierigkeiten bei Patienten mit davon differierenden Natriumspiegeln. Zudem berücksichtigt dieses Programm nicht die Unterschiede zwischen der Austauschleistung der einzelnen Dialysatoren, so daß auch hier Disäquilibriumserscheinungen nicht vermieden werden können.

Die Fachwelt hat die Bestimmung der Natriumelimination bei der Dialyse für nicht möglich gehalten, da bereits bei einem sehr niedrigen Meßfehler von 1 % über die Dauer der Dialyse eine hohe absolute Abweichung, d. h. Natriumverlust oder - zunahme, sich ergeben könne. Demzufolge wurde auf die Bestimmung der Natriumelimination bei der Dialyse verzichtet (vgl. H.G. Sieberth et al, « Aktuelle Probleme der Dialyseverfahren und der Niereninsuffizienz » 3. Symposium, Innsbruck 1969, S. 206-214, insbesondere S. 211, 3. Abs.).

Infolge dieser meßtechnischen Schwierigkeiten hat die Fachwelt generell mit bestimmten vorgegebenen Dialysierlösungszusammensetzungen operiert, die entweder konstant oder aber gemäß einem bestimmten Programm veränderlich (Natriummodelling) sind. Demzufolge wurde also bis heute der Patient zwangsweise einer bestimmten Dialysierlösungszusammensetzung unterworfen, nicht jedoch die Zusammensetzung dem Patienten angepaßt, da dies nicht für möglich gehalten worden ist.

Aus der EP-A-0 029 793 ist eine Vorrichtung der eingangs erwähnten Art bekannt, bei der ein erster Sensor intrakorporal in den Patienten und ein weiter Sensor extrakorporal stromauf eines Dialysators im Blutweg angeordnet ist. Desweiteren kann ein dritter Sensor stromab des Dialysators angeordnet sein, mit dem die Funktion des Dialysators überprüft werden kann. Zur Feststellung von Elektrolytunterschieden stromauf und stromab des Dialysators kann diese Anordnung nicht eingesetzt werden.

Der Erfindung liegt daher die Aufgabe zugrunde, eine Dialysiervorrichtung der eingangs erwähnten Art zur Verfügung zu stellen, mit der Unterschiede in der Elektrolytzusammensetzung der unbehandelten und behandelten, Dialysierflüssigkeit festgestellt und gegebenenfalls die Zusammensetzung der Dialysierflüssigkeit den Bedürfnissen des Patienten eingestellt werden kann.

Lösungen dieser Aufgabe erfolgen durch die kennzeichnenden Merkmale der Ansprüche 1, 2 und 5.

Überraschenderweise wurde nunmehr festgestellt, daß der Elektrolytgehalt der den Dialysator durchfließenden Dialysierlösung stromauf und stromab des Dialysators jeweils mit einem stromauf und stromab des Dialysators angeordneten Sensor derart genau bestimmt werden kann, daß hierdurch gegebenenfalls die Zusammensetzung der Dialysierlösung exakt den Bedürfnissen des Patienten durch entsprechende Regelung angepaßt werden kann.

Erfindungsgemäß ist zunächst eine Auswerteeinheit in Verbindung mit den beiden vorstehend genannten Sensoren vorgesehen, die in einer nachgeschalteten Differenziereinheit, d. h. Komparator, den Unterschied der Zusammensetzung des Elektrolytgehalts sowohl differenziell als auch integral für bestimmte, vorwählbare Zeiträume anzeigen kann. Demgemäß kann also der Unterschied des Elektrolytgehalts der den Dialysator durchfließenden Flüssigkeiten überwacht und integral für die Zeit der Dialysebehandlung festgehalten werden.

Gemäß einer berorzugten Auführungsform wird der von der Auswerte- und Differenziereinheit festgestellte Wert zur Regelung der Zusammensetzung der Dialysierflüssigkeit derart verwendet, daß der Elektrolythaushalt des Patienten auf den vom Arzt gewünschten Wert eingestellt wird. Man wird also regelmäßig die Zusammensetzung der Dialysierlösung so steuern, daß die Elektrolytzusammensetzung des Blutes des Patienten üblicherweise derjenigen eines gesunden, also nicht am Nierenversagen leidenden Menschen entspricht. Andererseits können jedoch auch spezielle, dem Patienten angepaßte elektrolytzusammensetzungen des Blutes hiermit eingestellt werden, bei denen sich der Patient üblicherweise gut fühlt, also keinen Stoffwechselstörungen unterzogen wird.

Die erfindungsgemäße Dialysiervorrichtung arbeitet auf folgende Weise :

Mit der erfindungsgemäßen Dialysiervorrichtung läßt sich zunächst bestimmungsgemäß die Elektrolytkonzentration der Dialysierlösung vorwählen, mit der der Patient zunächst behandelt wird. Eine derartige, zunächst eingestellte Dialsierlösung tritt in den Dialysator ein und wird im Dialysator zu Austauschzwecken entlang der Dialysemembran herangezogen. Weist nun diese Dialysierlösung einen Unterschied in der Konzentration der Elektrolyte im Vergleich zu Blut auf der Eintrittsseite auf, so wird dieser Konzentrationsunterschied in einem Dialysator mit hoher Austauschleistung bis zum Austritt des Blutes bis auf eine Differenz von etwa 5 % abgebaut. Diese letztgenannte Differenz ist auf die im Blut vorliegenden Plasma-Anionen zurückzuführen, die die semipermeable Membran weniger gut durchdringen können. Diese bleibende Differenz macht also etwa 5 % der Absolutkonzentration aus und wird durch die Gibbs-Donnan-Theorie erklärt.

Hieraus ist bereits ersichtlich, daß eine derartige Konzentrationsdifferenz in der Regel bei der Dialysebehandlung eines Patienten unerwünscht ist, da — wie vorstehend erläutert — starke Veränderungen des Elektrolythaushalts des Patienten zu den unerfreulichen Disäquilibriumserscheinungen führen.

Infolgedessen wird die Konzentration der Elektrolyte der Dialysierlösung am Ausgang des Dialysators mit dem zweiten Detektor gemessen, wobei die erhaltene und vorstehend erwähnte Konzentrationsdifferenz am Ausgang des Dialysators berücksichtigt wird. Dieser erhaltene Wert differiert demnach von dem am Dialysatoreingang erhaltenen Wert der Konzentration der Dialysierlösung, sofern ein Konzentrationsunterschied der Elektrolyte zwischen Dialysierlösung und Blut vorliegt. Demnach läßt sich durch Einspeisung dieser beiden Werte, die durch Messung der Elektrolytkonzentrationen stromauf und stromab des Dialysators erhalten werden, in die erfindungsgemäße Regeleinheit die Mischeinrichtung, insbesondere deren Pumpe steuern, mit der das Konzentrat in die Mischeinrichtung gefördert wird.

Somit kann erfindungsgemäß die Elektrolytkonzentration bei der Dialyse kontinuierlich bei einem bestimmten Wert oder aber entsprechend einer zeitlichen Abfolge von Werten geregelt werden, wobei diese Regelung direkt auf der im Blut vorhandenen Elektrolytkonzentration basiert. Diese Regelung hat den Vorteil, daß — anders als bei den bisher eingesetzten Regelungen — die Elektrolytkonzentration im Blut die Steuerungskonstante ist.

Vorteilhafterweise reicht es aus, daß zur Erzeugung der Dialysierflüssigkeit nur eine Konzentratlösung eingesetzt wird, die im Verhältnis von etwa 1 : 34 mit Leitungswasser verdünnt wird. In der Regel wird nämlich — wie bereits in der Einleitung erläutert — die Konzentration der Dialysierflüssigkeit bei der Dialysebehandlung allenfalls um etwa ± 8 % schwanken, so daß der Einfluß auf die übrigen Elektrolyte, beispielsweise Kalium oder Calcium, praktisch vernachlässigbar ist.

Andererseits können jedoch auch unterschiedliche Konzentrate mit unterschiedlichen Elektrolyten eingesetzt werden, wobei dann jeder Konzentratbehälter mit einer Pumpe verbunden ist, über die die bestimmten Konzentratmengen der Mischeinrichtung zugeführt werden. Die Pumpen selbst werden wiederum über Regeleinrichtungen geregelt, die mit ionenselektiven Sensoren oder Detektoren in Verbindung sind. Mit einem derartigen ionenselektiven Sensor kann die spezielle Konzentration eines bestimmten Elektrolyten bestimmt und beliebig und unabhängig von den übrigen Elektrolyten geregelt werden.

In einer weiteren Ausführungsform ist jeweils ein Sensor oder Detektor zur Bestimmung des gesamten Elektrolytgehalts stromauf und stromab des Dialysators vorgesehen. Vorteilhafterweise ist diese Anordnung der beiden Sensoren jeweils mit einem Temperatursensor verbunden, der benachbart angeordnet ist. Diese Temperatursensoren dienen zur Temperaturkompensation der ermittelten Werte, beispielsweise der elektrochemischen Potentiale oder des Leitfähigkeitswertes. Sofern jedoch die Temperatur der zugeführten Dialysierlösung im wesentlichen der Körpertemperatur des Patienten entspricht, kann diese Temperaturkompensation weggelassen werden.

Diese Sensoren sind mit einer Ist-Wert-Einrichtung verbunden, die wiederum mit einer vorprogrammierten Soll-Wert-Einrichtung verbunden ist. Sofern der Ist-Wert vom Soll-Wert abweicht, wird eine Korrektur der Zusammensetzung der Dialysierlösung dadurch vorgenommen, daß die die Konzentratlösung zur Mischeinrichtung fördernde Pumpe solange geregelt wird, bis der Ist-Wert mit dem Soll-Wert übereinstimmt.

Zur Bestimmung der Gesamtionenkonzentration können entweder die Leitfähigkeitsmessung oder aber die Bestimmung der Potentiale der Ionen, insbesondere des Natriumions, mittels ionenselektiver Elektroden vorteilhafterweise zum Einsatz kommen. Die letztgenannte Methode besitzt gegenüber der erstgenannten Methode den Vorteil, daß mehrere Ionenarten selektiv meßbar und mit Hilfe der erfindungsgemäßen Vorrichtung regelbar sind. Andererseits sind die zum Einsatz kommenden Elektroden wesentlich anfälliger und instabiler als die Ionenleitfähigkeitsmeßzelle, so daß man bei einer üblichen Dialyse der Leitfähigkeit den Vorzug geben wird.

Überdies zeigen ionenselektive Elektroden eine Potentialdrift, wenn sie unterschiedlichen Drücken, beispielsweise Unterdruck, ausgesetzt werden, der zur Erzeugung der Ultrafiltration auf der Seite der Dialysierflüssigkeit an den Dialysator angelegt wird. Gemäß einer weiteren Ausführungsform wird diesem Verhalten Rechnung getragen, wobei die Messung druckausgeglichen durchgeführt wird. Zu diesem Zweck sind jeweils stromauf und stromab des Dialysators von den Leitungen der Dialysierlösung abzweigende Leitungen vorgesehen, die mittels Absperrorganen absperrbar sind. Synchronisiert mit diesen Absperrorganen ist wenigstens eine Pumpe stromab vorgesehen, die den in der Dialysierlösungsleitung herrschenden Unterdruck überwindet. An diese Pumpe schließt sich die aus wenigstens einem Detektor bestehende Meßanordnung an. Gemäß dieser Ausführungsform wird alternierend gemessen, d. h. der Detektor wird wechselweise mit unbehandelter und behandelter Dialysierlösung beaufschlagt.

Weitere Merkmale, Einzelheiten und Ausführungsformen der Erfindung sind anhand der nachfolgenden Beschreibung unter Bezugnahme auf die Zeichnung erläutert.

Es zeigen :

Figur 1 eine schematische Darstellung einer ersten Ausführungsform einer Dialysiervorrichtung mit jeweils einem Detektor stromauf und stromab des Dialysators in der Dialysierlösungsleitung und

Figur 2 eine schematische Ansicht einer weiteren Ausführungsform mit jeweils einer Abzweigung in der Dialysierlösungsleitung stromauf und stromab des Dialysators, wobei die abzweigenden Leitungen zu einem Detektor führen.

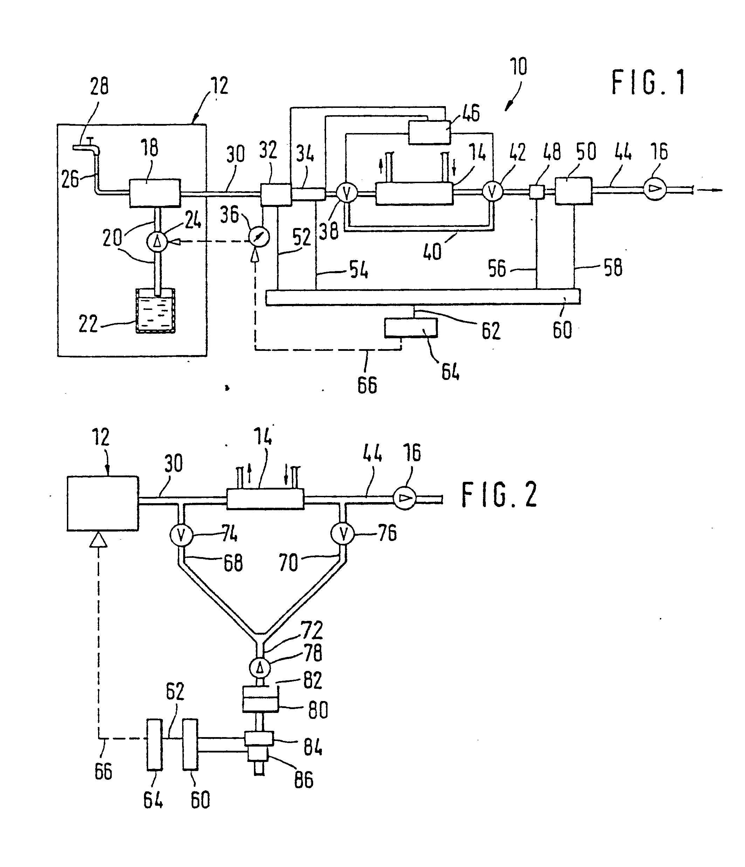

In Fig. 1 ist mit 10 eine Dialysiervorrichtung gezeigt. Diese Dialysiervorrichtung besteht im wesentlichen aus einer Einheit 12 zur Erzeugung der Dialysierlösung und einem Dialysator 14, der mit der Einheit 12 verbunden ist und an den sich stromab eine Pumpe 16 zur Erzeugung eines Unterdrucks im Dialysator auf der Seite der Dialysierflüssigkeit anschließt.

Die Einheit 12, die vereinfacht dargestellt ist, weist als Hauptbestandteil eine nicht näher erläuterte Mischeinrichtung 18 auf, die über eine Leitung 20 mit einem eine Konzentratlösung enthaltenden Behälter 22 in Verbindung steht. In dieser Leitung 20 ist eine steuerbare Pumpe 24 angeordnet, mit der die Konzentratlösung in die Mischeinrichtung gefördert werden kann.

Die Mischeinrichtung 18 steht weiterhin über eine Leitung 26 mit einer Frischwasserzuführung 28 in Verbindung. Das in der Mischeinrichtung 18 ankommende Wasser wird durch einen nicht gezeigten Heizblock auf etwa die Körpertemperatur des Patienten erwärmt. Anschließend saugt die Pumpe 24 Konzentrat aus dem Behälter 22 ab, das anschließend in der Mischeinrichtung mit dem erwärmten Leitungswasser vermischt wird.

In dieser Mischeinrichtung erfolgt weiterhin die Abtrennung von überschüssigem in der Dialysierlösung gelöstem Gas, das ansonsten im Dialysator 14 freigesetzt würde, da dort ein bestimmter Unterdruck vorliegt.

Von der Mischeinrichtung 18 geht eine Leitung 30 ab, über die die hergestellte Dialysierlösung zum Dialysator 14 gefördert wird. In dieser Leitung 30 ist ein erster Detektor 32 vorgesehen, mit dem wenigstens ein Konzentrationsparameter der in der Dialysierflüssigkeit enthaltenen Elektrolyte gemessen werden kan. Üblicherweise wird dies die Konzentration des Natriumsalzes sein, da dieses wenigstens 90 % des Leitfähigkeitswertes ausmacht. Vorzugsweise kan jedoch auch die Summe sämtlicher Konzentrationsparameter gemessen werden, da üblicherweise sämtliche Konzentrationen im gleichen Verhältnis zueinander vorliegen. Dies ist darauf zurückzuführen, daß nur eine Konzentratlösung vorgelegt wird.

Wie nachstehend erläutert wird, ist jedoch der Einsatz einer solchen Konzentratlösung, die sämtliche Elektrolyte im Gemisch enthält, nicht zwangsläufig notwendig. So ist es denkbar, daß hier das Elektrolytsalz in Form eines Konzentrats vorliegt und jeweils über ein Fördersystem, das im wesentlichen der Leitung 20 und der Pumpe 24 entspricht, dem Mischsystem 18 zugeführt wird. Vorteilhafterweise können ein Natriumsalz, insbesondere Natriumchlorid in Form seines Konzentrats und die übrigen Elektrolyte in einem weiteren Konzentrat vorliegen. Besonders bevorzugt ist jedoch der Einsatz eines bestimmten Konzentrats, wie in Fig. 1 dargestellt, da sich die Natriumionkonzentration in der Dialysierflüssigkeit allenfalls um maximal 10 % beim Dialysieren ändert, was natürlich auch eine relative Änderung gleicher Größe bei den übrigen Elektrolyten zur Folge hat, die jedoch im Organismus ohne größere Schwierigkeiten toleriert wird.

Im Detektor 32, der stromauf des Dialysators 14 angeordnet ist, erfolgt also die Messung eines Konzentrationsparameters der Dialysierlösung. Sofern eine Leitfähigkeitsmeßzelle als Detektor 32 eingesetzt wird, was ansich bevorzugt ist, erfolgt hier die Messung der Leitfähigkeit der gesamten Dialysierlösung. Der erhaltene Meßwert wird mit Hilfe eines sich an den Detektor 32 anschließenden Temperaturdetektor 34 kompensiert. Der Detektor 32 steht weiterhin mit einer Steuereinheit 36 in Verbindung, die die Pumpe 24 entsprechend dem im Detektor 32 festgestellten Meßwert steuern kann.

An dem Temperaturdetektor 34 schließt sich ein in der Leitung 30 angeordnetes Bypass-Ventil 38 an, von dem einerseits die Leitung 30 zum Dialysator 14 weitergeht und andererseits eine Bypass-Leitung 40 abzweigt. Diese Bypass-Leitung 40 ist mit einem weiteren Bypass-Ventil 42 in Verbindung, das stromab des Dialysators in der Leitung 44 angeordnet ist. Beide Bypass-Ventile sind elektrisch mit einer Steuereinrichtung 46 verbunden, die ebenfalls elektrisch mit dem Detektor 32 und dem Temperaturdetektor 34 verbunden ist. Weicht die Temperatur oder der im Detektor 32 gemessene Meßwert vom Soll-Wert ab, so steuert die Steuereinheit 46 die Bypass-Ventile derart, daß die noch nicht den gewünschten Bedingungen entsprechende Dialysierflüssigkeit durch die Bypass-Leitung 40 Dialysator 14 vorbeigeleitet wird. Hierdurch wird vermieden, daß Dialysierflüssigkeit falscher Zusammensetzung oder Temperatur zum Dialysator gelangt. Ist jedoch die Zusammensetzung und die Temperatur der Dialysierflüssigkeit korrekt, so gelangt diese zum Dialysator 14 und anschließend durch einen weiteren Temperaturdetektor 48 und Detektor 50, mit dem wiederum wenigstens ein Konzentrationsparameter in der Dialysierflüssigkeit gemessen werden kann. Diese Detektoren sind, wie gesagt, stromab des Dialysators 14 in der Leitung 44 angeordnet. An diese Detektoren 48 und 50 schließt sich die Pumpe 16 an, die in dem von der Einheit 12 bis zur Pumpe 16 reichenden Leitungssystem, in dem die Dialysierflüssigkeit gefördert wird, einen bestimmten Unterdruck anlegt, der zur Steuerung der Ultrafiltration eingesetzt wird.

Die Detektoren 32, 34, 48 und 50 sind jeweils über die Leitungen 52, 54, 56 und 58 mit einer Auswertungseinheit 60 verbunden, an die sich über eine Leitung 62 eine Differenziereinheit 64 anschließt. Von dieser Differenziereinheit 64 geht, wie mit 66 liniert angedeutet ist, ein Signal an die Steuereinheit 36 ab, sofern sich in der Differenziereinheit 64 eine Differenz ergibt, die vom eingestellten Soll-Wert abweicht.

Die in Fig. 1 dargestellte Ausführungsform weist folgende Arbeitsweise auf :

In der Einheit 12 wird zunächst eine Dialysierlösung auf übliche Weise hergestellt. Wenn diese Dialysierlösung die Einheit 12 verläßt, sind die Bypass-Ventile 38 und 42 auf Umleitung geschaltet, und zwar so lange, bis der Detektor 32 den in ihm fest eingestellten Konzentrationswert anzeigt, der jedoch durch die übergeordnete Differenziereinheit 64 verändert werden kann.

Ist die gewünschte Dialysierlösung hergestellt, so wird diese mittels der Pumpe 16 durch den Dialysator 14 unter Erzeugung eines Unterdrucks gefördert, wobei natürlich die Bypass-Ventile 38 und 42 umgeschaltet sind. Hier setzt nun die erfindungsgemäße Steuerung des Gehalts der Dialysierlösung ein. Sofern der Detektor 50 ein Signal an die Auswerteeinheit und darauf folgend an die Differenziereinheit 64 abgibt, das um einen bestimmten Betrag gegenüber dem von dem Detektor 32 abgegebenen Signal abweicht, also ein Differenzwert gebildet wird, der von dem in der Differenziereinheit 64 festgelegten Wert abweicht, steuert diese Differenziereinheit 64 die Steuereinheit 36, wie mit 66 gezeigt, an, die wiederum die Pumpe 24 in Betrieb setzt oder ausschaltet, je nachdem ob eine höher oder niedriger konzentrierte Dialysierlösung erzeugt werden soll.

Dabei wird die Differenz in der Differenziereinheit 64 so gewählt, daß der Konzentrationsunterschied der in der Dialysierflüssigkeit enthaltenen Natriumionen stromauf und stromab des Dialysators nicht über 5 mmol/l, vorzugsweise nicht über 1-2 mmol/l und insbesondere bei etwa 0 mmol/l liegt. Sofern nämlich kein Differenzbetrag stromauf und stromab des Dialysators festgestellt wird, weist das aus dem Blut durch den Dialysator 14 abgezogene Ultrafiltrat praktisch die gleiche Elektrolytzusammensetzung wie das Blut selbst auf, was im wesentlichen angestrebt wird.

In einer Weiterentwicklung dieser in Fig. 1 gezeigten Ausführungsform können die Bypass-Ventile 38 und 42, sowie die Bypass-Leitung 40 zur Überprüfung der Detektoren 32, 34, 48 und 50 herangezogen werden. Dabei werden jeweils die Detektoren 32 und 50, sowie 34 und 48, miteinander dadurch verglichen, daß reine Dialysierlösung durch die Leitung 30, die Leitung 40 und die Leitung 44 am Dialysator 14 vorbeigepumpt wird. In diesem Überprüfungszustand, der regelmäßig, beispielsweise etwa alle 10-15 Minuten, durchgeführt wird, werden die jeweiligen Werte der Detektoren in der Auswertungseinheit 60 auf Null gestellt, so daß sich eine absolute Eichung der eingesetzten Detektoren erübrigt und lediglich eine identische Konzentrationsabhängigkeit der Detektoren existieren muß.

Nach dem Eichen, d. h. nach entsprechender Umschaltung der Bypass-Ventile 38 und 42 erfolgt wieder die übliche Dialysierung des Patienten. Die von der Pumpe 16 abgepumpte Dialysierflüssigkeit wird danach in den Abfluß geleitet.

Als Detektoren 32 und 50 eignen sich sämtliche Detektoren, die zur Bestimmung von Ionenkonzentrationen in Flüssigkeiten herangezogen werden können. Hierzu gehören die Leitfähigkeitsmessung, die elektrochemische Messung einzelner Ionenarten oder der Summe sämtlicher Ionen, spektrographische Messung, magnetische Messung und dergleichen. Zu den bevorzugt eingesetzten Detektoren gehören die Leitfähigkeitsmeßzelle und ionenselektive Elektroden.

Der Einsatz der Leitfähigkeitsmeßzelle ist bei den heute üblichen Dialysiervorrichtungen bekannt. So wird eine Leitfähigkeitsmeßzelle zur Überwachung des einzustellenden Leitfähigkeitswertes stromauf des Dialysators eingesetzt, wobei sie lediglich zu Überwachungszweckes eines einmal eingestellten Wertes eingesetzt wird. Eine Regelung dieses Wertes ist im Stand der Technik allenfalls über das vorstehend erwähnte Natriummodelling vorgesehen, das sich nicht an den jeweiligen Verhältnissen ausrichtet, die sich aus der Elektrolytkonzentration im Patienten und den eingesetzten Dialysatoren ergeben. Ionenselektive Elektroden sind ebenfalls bekannt, beispielsweise aus Cammann, Das Arbeiten mit ionenselektiven Elektroden, 2. Aufl. 1977, Springer-Verlag, Berlin, und and aus DE-A-2215378. Derartige ionenselektive Elektroden werden aus einem Ionaustauschermaterial hergestellt, das entweder kationisch oder anionisch aktiv ist. Zu solchen Materialien gehören beispielsweise quartanäre Ammoniumgruppen, Phosphoniumionen, oder Sulfoniumionen, die beispielsweise organische Radikale aufweisen können. Ferner sind langkettige aliphatische Markaptane, alkylierte Phenole oder makrozyklische Äther, beispielsweise Kronenäther, einsetzbar. Insbesondere ist es möglich, Membranelektroden zu schaffen, welche auf Alkaliionen ansprechen und Komplexe von Kronenäther oder analogen Verbindungen, insbesondere des Valinomycins, enthalten.

Weiterhin lassen sich auch 2- oder mehrwertige Metallionen bestimmen, so daß praktisch jedes mögliche Metallion gemessen werden kann.

Zu weiteren kationenempfindlichen Materialien gehören Metallchelate oder Ionenaustauschsalze oder Ionenaustauschmaterialien. Besonders bevorzugt ist für die Messung von Kaliumionen Valinomycin, für Calciumionen ebenfalls ein Dioxakorksäurediamidderivat, das sich von dem vorstehenden Diamid unterscheidet. Andererseits können Natriumionen auch mit einem Na-selektiven Glas bestimmt werden, das gegenüber ionenselektiven Elektroden auf organischer Basis wegen seiner Ünempfindlichkeit bevorzugt ist. Auch der pH-Wert kann mit einer pH-selektiven Glaselektrode bestimmt werden, die ebenfalls handelsüblich ist und beispielsweise in der DE-A-2134101 beschrieben ist.

Ionenselektive Elektroden auf organischer Basis werden üblicherweise in Form von dünnen Membranen aus PVC-Material hergestellt, dem ein Weichmacher zugesetzt wird. Derartige polymere Materialien und die Herstellung sowie die Zugabe von Weichmachern sind beispielsweise in der DE-A-2215378 beschrieben.

Eine derart hergestellte ionenselektive Elektrode wird mittels der üblichen Ableitung, beispielsweise einer Elektrolytlösung als Stromschlüssel (gesättigte KCl-Lösung) mit einer Ableitelektrode verbunden, die ihrerseits mit einem üblicherweise eingesetzten Meß- und Verstärkergerät in Verbindung ist, das erfindungsgemäß als Auswertungseinheit 60 bezeichnet ist.

Gemäß dem in Fig. 1 gezeigten Ausführungsbeispiel werden vorzugsweise Detektoren 32 und 50 gleicher Zusammensetzung eingesetzt, also entweder Leitfähigkeitsmeßzellen oder aber ionenselektive Elektroden.

Infolge der konstanten Zusammensetzung der Konzentratlösung, die im Behälter 22 enthalten ist, kann über eine Natrium-selektive Elektrode die Gesamtzusammensetzung der Dialysierflüssigkeit gesteuert werden.

In Fig. 2 ist eine weitere Ausführungsform der Erfindung gezeigt, wobei gleiche Bezugszeichen für gleiche Teile verwendet werden. Diese Ausführungsform weist wiederum eine Einheit 12 zur Erzeugung der Dialysierflüssigkeit auf. Von dieser Einheit 12 geht die Leitung 30 ab, die direkt mit dem Dialysator verbunden ist, an den sich die Leitung 44 anschließt. Die Leitungen 30 und 44 weisen je eine abzweigende Leitung 68 und 70 auf, die sich zu einer Leitung 72 vereinigen. Vorteilhafterweise ist die Leitung 68 unmittelbar stromauf und die Leitung 70 unmittelbar stromab des Dialysators 14 angeordnet.

Da in den Dialysierlösungsleitungen 30 und 44 durch die Pumpe 16 ein Unterdruck erzeugt wird, sind die Leitungen 68 und 70 durch Absperrorgane 74 und 76 abgesperrt und werden vorteilhafterweise alternierend geöffnet und geschlossen. Um frische oder verbrauchte Dialysierlösung in die Leitung 72 zu saugen, ist in dieser Leitung 72 eine Pumpe 78 vorgesehen, die den Unterdruck in den Leitungen 30 und 44 überwindet. An diese Pumpe 78 schließt sich ein Vorratsgefäß 80 an, in dem ein Druckausgleich stattfindet, beispielsweise durch eine im Vorratsgefäß 80 vorgesehene Öffnung 82. Stromab dieses Vorratsgefäßes ist der Detektor 84 vorgesehen, der in seiner Funktion und seinem Aufbau den Detektoren 32 und 50 entspricht und der durch einen Temperaturdetektor 86 temperaturkompensiert wird. An diese Detektoren 84 und 86 schließt sich wiederum die Auswertungseinheit 60 mit der üblichen Steuerung an. Wie aus dem in Fig. 2 gezeigten Ausführungsbeispiel ersichtlich, arbeitet diese Ausführungsform nur mit einem Detektor, der alternierend mit frischer oder verbrauchter Dialysierlösung beaufschlagt werden kann, und kommt somit mit einem Detektor aus. Auch diese Ausführungsform wird als unter die Erfindung fallend betrachtet. Allerdings ist zur stetigen Kontrolle der Gesamtzusammensetzung der Dialysierlösung kein weiterer Detektor mehr in der unmittelbar zum Dialysator 14 führenden Leitung 30 nötig. Vorzugsweise weist diese Leitung 30 jedoch einen derartigen Detektor auf, der dem in Fig. 1 gezeigten Detektor 32 entspricht, insbesondere eine Leitfähigkeitsmeßzelle, mit der sofort starke Schwankungen in der Dialysierlösung festgestellt werden können, so daß die Dialysevorrichtung unterbrochen werden kann.

Gemäß dieser bevorzugten Ausführungsform kann also in der Leitung 30 eine Leitfähigkeitsmeßzelle vorgesehen sein, während als Detektor 84 eine ionenselektive Elektrode in Betracht kommen kann, die durch den alternierenden Betrieb stets mit frischer Dialysierlösung geeicht wird und lediglich die Differenz der durch den Leitungszweig 70 geförderten verbrauchten Dialysierlösung bezogen auf die frische Dialysierlösung feststellen muß. Eine solche in Fig. 2 gezeigte Ausführungsform hat den Vorteil, daß die üblichen heute eingesetzten Dialysiervorrichtungen mit einer extern vorgesehenen Detektorvorrichtung, insbesondere einer ionenselektiven Meßanordnung, verbunden werden können, wobei lediglich das mit dem Dialysator 14 in Verbindung stehende Schlauchsystem zwei Anschlüsse aufweisen muß, die die Leitungsverbindung zu den Leitungen 68 und 70 herstellen. Andererseits kann natürlich auch als Detektor 84 eine übliche Leitfähigkeitsmeßzelle vorgesehen sein.

In einer weiter bevorzugten Ausführungsform hat sich herausgestellt, daß man vorteilhafterweise mit einem Detektor 32 und einer Steuereinheit 36, die die Pumpe 24 und damit den Konzentratfluß steuert, zunächst die untere, noch vom Patienten tolerierte Elektrolytzusammensetzung herstellt und überwacht, also beispielsweise eine Dialysierflüssigkeit mit einem Natriumgehalt von 135 mmol/l. In dieser Ausführungsform ist vorgesehen, daß aus dem Behälter 22 eine weitere der Leitung 20 entsprechenden Leitung und eine weitere der Pumpe 24 entsprechende Pumpe zur Feinregulierung der Zusammensetzung der Dialysierlösung angeordnet ist. Diese weitere Pumpe wird von einer ebenfalls der Steuereinheit 36 entsprechenden Einheit und der Differenziereinheit 64 gesteuert. Diese Ausführung hat den Vorteil, daß die Überwachung und die Feinregulierung der Dialysierlösung sich nicht überlagern und von einander getrennt sind. In diesem Fall kann der Detektor 32 entweder allein oder es kann ein weiterer Detektor stromauf des Dialysators 14 vorgesehen sein, der gleiche oder unterschiedliche Eigenschaften wie der Detektor 32 besitzt.

Die vorstehende Beschreibung bezieht sich auf eine Dialysiervorrichtung, die unter den Begriff « Vorrichtung zum Reinigen von Blut » fällt. Insofern ist natürlich eine derartige Anwendung der Erfindung nicht nur auf das Dialysieren beschränkt, sondern erstreckt sich auch auf andere Vorrichtungen zum Reinigen von Blut, beispielsweise auf die Hämofiltration. Bei der Hämofiltration wird Plasma in einem Hämofilter von den quasi festen Bestandteilen des Bluts abfiltriert. In diesem Fall entspricht der eingesetzte Hämofilter dem vorstehend erwähnten Dialysator 14. Bei der Hämofiltration wird die Substituatlösung stromab des Hämofilters entsprechend der entzogenen Plasmamenge dem Blut wieder zugesetzt. In diesem Fall werden entsprechend der in Fig. 1 bis 4 gezeigten Anordnung ein Detektor am Bluteingang und ein Detektor am Blutausgang des Hämofilters sowie ein Detektor am Plasmaauslaß entsprechend einer ersten Ausführungsform vorgesehen. Die Zumischung des Substituats erfolgt wiederum in Form eines Konzentrats, das zur Erstellung des Substituats herangezogen wird. Anstelle eines derartigen Konzentrats können natürlich auch fertige Lösungen eingesetzt werden, deren Zusammensetzung dem unteren vom Patienten tolerierten Wert entspricht. Es erfolgt dann wiederum eine aus einem Konzentrat gebildete Feinregulierung dieser Zusammensetzung nach oben wobei, beispielsweise die Differenz der Meßwerte am Bluteingang und Blutausgang oder aber der absolute Meßwert am Plasmaauslaß gemessen werden. Andererseits können jedoch auch Bluteingang und Plasmaauslaß hinsichtlich ihrer Meßwerte miteinander verglichen werden.

Mit einer derartigen Hämofiltrationsanordnung ist es möglich, im Direktbetrieb die Zusammensetzung der Substituatlösung entsprechend den bei der Hämofiltration vorliegenden Bedingungen abzuändern und anzupassen.

Hinzuzufügen ist noch, daß jede dieser vorstehend erläuterten Vorrichtungen zum Reinigen von Blut bis zu vier Meßpunkte aufweisen kann, die mit wenigstens einem Detektor verbunden sind. In einer solchen Ausführungsform werden die Meßstellen nach einer bestimmten Schaltungsmethode nacheinander abgegriffen und in einer oder mehrerer Differenziereinheiten ausgewertet.

Weiterhin ist noch anzumerken, daß die Auswertungseinheit 60 und gegebenenfalls die Differenziereinheit 64 zur Messung der Elektrolytkonzentrationen der unbehandelten und behandelten, durch den Dialysator geführten Flüssigkeiten herangezogen werden können. So kann beispielsweise die Elektrolytkonzentration am Eingang und am Ausgang des Dialysators 14 direkt gemessen und die Differenz dieser Werte sowohl differenziell als auch über einen bestimmten Zeitraum integral bestimmt und angezeigt werden.

Gemäß dieser Ausführungsform ist also noch keine Veränderung der Zusammensetzung der Dialysierlösung durch entsprechende Steuerung der Einheit 12 zur Erzeugung der Dialysierlösung vorgesehen.

Somit ist diese Ausführungsform eine reine Meßvorrichtung zur Messung und zur Differenziellen und/oder integralen Anzeige der Elektrolytbilanz, insbesondere der Natriumbilanz.

**Patentansprüche**

1. Vorrichtung zum Reinigen von Blut mit einem Dialysator (14), der durch eine Membran in einen Blutweg und einen Dialysierflüssigkeitsweg geteilt ist, mit einer Einheit (12) zur Erzeugung der Dialysierflüssigkeit, bei der eine Mischeinrichtung (18) mit wenigstens einem Konzentratbehälter (22), wenigstens einer damit verbundenen Konzentratpumpe (24) und einer Frischwasserquelle (28) verbunden ist, mit Pumpen (16, 96, 108) zur Förderung des Blutes und der Dialysierflüssigkeit, mit einer Ultrafiltrationseinheit, mit jeweils einem identischen Sensor (32, 50) stromauf und stromab des Dialysators (14) sowie mit einer Auswerteeinheit (60) und einer Anzeigeeinheit für die Signale der Sensoren (32, 50), dadurch gekennzeichnet, daß die Sensoren (32, 50) im Dialysierflüssigkeitsweg angeordnet sind und Sensoren zur Bestimmung wenigstens des Natriumionengehaltes der Dialysierflüssigkeit sind und daß an die Auswerteeinheit (60) eine Einheit (64) zur Bildung eines Differenzwertes aus den stromauf bzw. stromab des Dialysators (14) im Dialysierflüssigkeitsweg gewonnenen Signalen angeschlossen ist.

2. Vorrichtung zum Reinigen von Blut mit einem Dialysator (14), der durch eine Membran in einen Blutweg und einen Flüssigkeitsweg geteilt ist, mit einer Einheit (12) zur Erzeugung der Dialysierflüssigkeit, bei der eine Mischeinrichtung (18) mit wenigstens einem Konzentratbehälter (22), wenigstens einer damit verbunden Konzentratpunpe (24) und einer Frischwasserquelle (28) verbunden ist, mit Pumpen (16, 96, 108) zur Förderung des Blutes und der Dialysierflüssigkeit, mit einer Ultrafiltrationseinheit, mit wenigstens einem Sensor (84) im Bereich des Dialysators (14) sowie mit einer Auswerteeinheit (60) und einer Anzeigeeinheit für das Signal des Sensors (84), dadurch gekennzeichnet, daß der Sensor (84) ein Sensor zur Bestimmung wenigstens des Natriumionengehaltes der Dialysierflüssigkeit ist und in eine Leitung (72) eingeschaltet ist, die sich in eine zweite Leitung (68), die mit dem Dialysierflüssigkeitsweg stromauf des Dialysators verbunden ist, und in eine dritte Leitung (70) verzweigt, die mit dem Dialysierflüssigkeitsweg stromab des Dialysators (14) verbunden ist, daß in die zweite Leitung (68) ein erstes Absperrorgan (74) und in die dritte Leitung (70) ein zweites Absperrorgan (76) eingeschaltet ist, die wechselweise gleichzeitig betätigbar sind, daß eine Pumpe (78) zur Förderung der Dialysierflüssigkeit vom Sensor (84) vorgesehen ist und daß an die Auswerteeinheit (60) eine Einheit (64) zur Bildung eines Differenzwertes entsprechend den stromauf bzw. stromab des Dialysators in Dialysierflüssigkeit gewonnenen Signalen angeschlossen ist.

3. Vorrichtung nach Anspruch 1 oder 2, dadurch gekennzeichnet, daß die Einheit (64) zur Bildung des Differenzwertes mit einer Regeleinheit (36) verbunden ist, die in der Einheit (12) zur Erzeugung der Dialysierflüssigkeit die Zusammensetzung der Dialysierflüssigkeit regelt.

4. Vorrichtung nach Anspruch 1, dadurch gekennzeichnet, daß zwischen dem ersten Sensor (32) und dem Dialysator (14) ein erstes Bypassventil (38) und zwischen dem zweiten Sensor (50) und dem Dialysator (14) ein zweites Bypassventil vorgesehen ist und die Bypassventile (38 und 42) mit einer Bypassleitung (40) verbunden sind und daß durch Betätigung der Bypassventile (38, 42) der erste und der zweite Sensor (32, 50) abgleichbar sind.

5. Hämofiltrationsvorrichtung mit einem Hämofilter, der durch eine Membran in eine mit Blut beaufschlagbare Kammer, in die der Blutweg geschaltet ist und in eine Plasmaabzugskammer geteilt ist, mit einer Einheit zur Bereitstellung von Substitutionsflüssigkeit, die über eine Substituatleitung mit dem Blutweg verbunden ist, mit Pumpen zur Förderung der Substituatlösung und des Blutes sowie zum Abfördern von Plasma sowie mit einer Bilanziereinheit, mit der die dem Blut abgezogene Flüssigkeit und die Substitutionsflüssigkeit gegeneinander bilanziert werden, dadurch gekennzeichnet, daß in der von der Plasmaabzugskammer abgehende Leitung ein erster Sensor und in der Substituatleitung in zweiter Sensor vorgesehen sind und die beiden Sensoren wenigstens den Natriumionengehalt des abgezogenen Plasmas bzw. der Substitutionsflüssigkeit bestimmen, daß die Signale der beiden Sensoren von einer Auswerteeinheit angezeigt werden, daß die Auswerteeinheit mit einer Einheit zur Bildung eines Differenzwertes zwischen den beiden Signalen verbunden ist und daß die Einheit zur Bildung eines Differenzwertes mit einer Regeleinheit verbunden ist, die bei Feststellung eines Differenzwertes die Einheit zur Erzeugung der Substitutionsflüssigkeit so lange regelt, bis ein vorbestimmter Wert erreicht ist.

6. Vorrichtung nach Anspruch 2, dadurch gekennzeichnet, daß stromauf des Sensors (84) in der Leitung (72) ein Vorratsgefäß (80) vorgesehen ist, das über eine Öffnung (82) belüftbar ist.

7. Vorrichtung nach einem der Ansprüche 1 bis 6, dadurch gekennzeichnet, daß der Sensor (32, 50, 84) im Dialysierflüssigkeitsweg, der Plasmaabzugsleitung oder der Substituatleitung eine Leitfähigkeitsmeßzelle oder wenigstens eine ionenselektive Elektrode ist.

8. Vorrichtung nach Anspruch 7, dadurch gekennzeichnet, daß die ionenselektive Elektrode eine natriumselektive, kaliumselektive, pH-selektive $CO_2$—, $HCO_3$-sensitive und/oder calciumselektive Elektrode ist.

9. Vorrichtung nach Anspruch 3, dadurch gekennzeichnet, daß von dem Konzentratbehälter (22) eine zweite Leitung abgeht, in die eine zweite Konzentratpumpe eingeschaltet ist, daß die erste Konzentratpumpe (24) von einer Steuereinheit gesteuert eine erste Konzentratmenge fördert, die zu einer unteren, noch vom Patienten tolerierten Dialysierflüssigkeitszusammensetzung führt und daß die zweite Konzentratpumpe von der Regeleinheit (36) auf ein Differenzsignal der Differenziereinheit (64) hin geregelt wird.

## Claims

1. Apparatus for purification of blood, comprising a dialyzer (14) being divided by a membrane into a blood path and a dialysis liquid path, comprising further a unit (12) for producing the dialysis liquid wherein a mixing unit (18) is combined with at least one concentrate container (22), at least one concentrate pump (24) connected thereto and a fresh-water source (28), further comprising pumps (16, 96, 108) for conveying the blood and the dialysis liquid, comprising an ultrafiltration unit, an identical sensor (32, 50) each upstream and downstream of the dialyzer (14), respectively, as well as an evaluation unit (60) and a display unit for the signals of the sensors (32, 50), characterized in that the sensors (32, 50) are arranged within the dialysis liquid path and are sensors for determining at least the sodium ion content of the dialysis liquid, and that a unit (64) for forming a difference value of the signals obtained upstream, and downstream of the dialyzer (14) respectively in the dialysis liquid path is connected to the evaluation unit (60).

2. Apparatus for purification of blood, comprising a dialyzer (14) being divided by a membrane into a blood path and a dialysis liquid path, comprising further a unit (12) for producing the dialysis liquid wherein a mixing unit (18) is combined with at least one concentrate container (22), at least one concentrate pump (24) connected thereto and a fresh-water source (28), further comprising pumps (16, 96, 108) for conveying the blood and the dialysis liquid, comprising an ultrafiltration unit, at least one sensor (84) adjacent to the dialyzer (14) as well as an evaluation unit (60) and an display unit for the signal of the sensor (84), characterized in that the sensor (84) is a sensor for determination of at least the sodium ion content of the dialysis liquid and is connected into a line (72) which branches into a second line (68) being connected with the dialysis liquid path upstream of the dialyzer and a third line (70) being connected with the dialysis liquid path downstream of the dialyzer (14), that into the second line (68) a first shut-off device (74) and into the third line (70) a second shut-off device (76) is connected which alternatingly are operable simultaneously, that a pump (78) for conveying the dialysis liquid from sensor (84) is provided and that a unit (64) for forming a difference value corresponding to the signals obtained upstream and downstream of the dialyzer, respectively, within the dialysis liquid is connected to the evaluation unit (69).

3. Apparatus according to claim 1 or 2, characterized in that the unit (64) for forming the difference value is connected to a control device (36) which controls the composition of the dialysis liquid in the unit (12) for producing the dialysis liquid controls.

4. Apparatus according to claim 1, characterized in that between the first sensor (32) and the dialyzer (14) a first bypass valve (38) and between the second sensor (50) and the dialyzer (14) a second bypass valve is provided and that the bypass valves (38 and 42) are connected with a bypass line (40) and that by operating the bypass valves (38, 42) the first and the second sensor (32, 50) may be calibrated.

5. Hemofiltration device comprising a hemofilter which is devided by a membrane into a chamber being loadable with blood and into which the blood path is connected, and a plasma discharge chamber, further comprising a unit for supplying substitution liquid which is connected with the blood path via a substituate line, further comprising pumps for conveying the substitution solution and the blood as well as for drawing off plasma and comprising a balancing unit with which the liquid drawn off the blood and the substitution liquid are balanced against each other, characterized in the line going off the plasma discharge chamber a first sensor and in the subtituate line a second sensor are provided and the two sensors determine at least the sodium ion content of the drawn off plasma and of the substitution liquid, respectively, that the signals of the two sensors are displayed by an evaluation unit, that the evaluation unit is connected with a unit for forming a difference value between the two signals and that the unit for forming a difference value is connected with a control unit which controls the unit for producing the substitution liquid upon ascertaining a difference value such long until a predetermined value is reached.

6. Apparatus according to claim 2, characterized in that upstream of the sensor (84) a tank (80) is provided in the line (72) which tank may be aerated via an aperture (82).

7. Apparatus according to claims 1 to 6, characterized in that the sensor (32, 50, 84) in the dialysis liquid path, the plasma discharge line or the substituate line is a conductivity measuring cell or at least an ion-selective electrode.

8. Apparatus according to claim 7, characterized in that the ion-selective electrode is a sodium-selective, potassium-selective, pH-selective, $CO_2$—, $HCO_3$-sensitive and/or calciumselective electrode.

9. Apparatus according to claim 3, characterized in that a second line goes off the concentrate container (22) into which line a second concentrate pump is connected, that the first concentrate pump (24) being controlled by a control unit conveys a first concentrate amount which leads to a minimal dialysis liquid composition, still tolerated by the patient, and that the second concentrate pump is controlled by the control unit (36) responsive to a difference signal of the difference unit (64).

## Revendications

1. Dispositif de purification du sang avec un dialyseur (14), qui est divisé par une membrane en un circuit sanguin et un circuit de liquide de dialyse, avec une unité (12) de production du liquide de dialyse, dans lequel est fixé un dispositif de mélange (18) avec au moins un réservoir de concentré (22), au moins une pompe à concentré qui y est reliée (24) et une source d'eau fraîche (28), avec des pompes (16, 96, 108) pour transporter le sang et le liquide de dialyse, avec une unité d'ultrafiltration, avec un senseur à chaque fois identique (32, 50) en amont et en aval du dialyseur (14) ainsi qu'avec une unité d'évaluation (60) et une unité d'affichage pour les signaux des senseurs (32, 50), caractérisé en ce que les senseurs (32, 50) sont disposés dans le circuit du liquide de dialyse et en ce qu'il s'agit de senseurs pour déterminer au moins la teneur en ions sodium du liquide de dialyse, et en ce qu'à l'unité d'évaluation (60) est jointe une unité (64) pour la formation d'une valeur différentielle à partir des signaux obtenus en amont ou en aval du dialyseur (14) dans le circuit du liquide de dialyse.

2. Dispositif de purification du sang avec un dialyseur (14), qui est divisé par une membrane en un circuit sanguin et un circuit de liquide, avec une unité (12) pour produire le liquide de dialyse, auquel est fixé un dispositif de mélange (18) avec au moins un réservoir de concentré (22), au moins une pompe à concentré qui y est liée (24) et une source d'eau fraîche (28), avec des pompes (16, 96, 108) pour transporter le sang et le liquide de dialyse, avec une unité d'ultrafiltration, avec au moins un senseur (84) dans la région du dialyseur (14) ainsi qu'avec une unité d'évaluation (60) et une unité d'affichage pour le signal du senseur (84), caractérisé en ce que le senseur (84) est un senseur pour déterminer au moins la teneur en ions sodium du liquide de dialyse et est intercalé dans une canalisation (72) qui se ramifie en une seconde canalisation (68), laquelle est reliée au circuit de liquide de dialyse en amont du dialyseur, et en une troisième canalisation (70), laquelle est reliée au circuit de liquide de dialyse en aval du dialyseur (14), en ce que dans la seconde canalisation (68) est intercalé un premier organe d'arrêt (74) et dans la troisième canalisation (70) un deuxième organe d'arrêt (76), que l'on peut actionner alternativement de façon simultanée, en ce qu'une pompe (78) est prévue pour faire circuler le liquide de dialyse du senseur (84) et en ce qu'à l'unité d'évaluation (60) est jointe une unité (64) pour la formation d'une valeur différentielle correspondant aux signaux obtenus en amont ou en aval du dialyseur dans le liquide de dialyse.

3. Dispositif selon les revendications 1 ou 2, caractérisé en ce que l'unité (64) pour la formation d'une valeur différentielle est reliée à une unité de réglage (36) qui dans l'unité (12) de production du liquide de dialyse règle la composition du liquide de dialyse.

4. Dispositif selon la revendication 1, caractérisé en ce qu'entre le premier senseur (32) et le dialyseur (14) est prévue une première soupape de dérivation (38) et entre le deuxième senseur (50) et le dialyseur est prévue une seconde soupape de dérivation et les soupapes de dérivation (38 et 42) sont reliées à une canalisation de dérivation (40) et en ce qu'en actionnant les soupapes de dérivation (38, 42) on peut régler les premier et second senseurs (32, 50).

5. Dispositif d'hémofiltration avec un hémofiltre, qui est divisé par une membrane en une chambre pouvant être alimentée en sang, dans laquelle est monté le circuit sanguin, et en une chambre d'extraction du plasma, avec une unité de préparation de liquide.de substitution, qui est reliée par l'intermédiaire d'une canalisation de produit de substitution au circuit sanguin, avec des pompes pour faire circuler la solution de produit de substitution et le sang ainsi que pour soutirer le plasma, ainsi qu'avec une unité d'équilibrage, avec laquelle on équilibre l'un par rapport à l'autre le liquide prélevé dans le sang et le liquide de substitution, caractérisé en ce que dans la canalisation provenant de la chambre d'extraction du plasma est prévu un premier senseur et dans la canalisation de produit de substitution un second senseur, et en ce que les deux senseurs déterminent au moins la teneur en ions sodium du plasma extrait ou du liquide de substitution, en ce que les signaux des deux senseurs sont indiqués par une unité d'évaluation, en ce que l'unité d'évaluation est reliée à une unité de formation d'une valeur différentielle entre les deux signaux et en ce que l'unité de formation d'une valeur différentielle est reliée à une unité de réglage, qui lors de l'établissement d'une valeur différentielle règle l'unité de production du liquide de substitution jusqu'à ce qu'une valeur prédéterminée soit atteinte.

6. Dispositif selon la revendication 2, caractérisé en ce qu'en amont du senseur (84) est prévu dans la canalisation (72) un réservoir (80), qui peut être aéré par une ouverture (82).

7. Dispositif selon l'une des revendications 1 à 6, caractérisé en ce que le senseur (32, 50, 84) dans le circuit de liquide de dialyse, la canalisation d'extraction de plasma ou la canalisation de produit de substitution est une cellule de mesure de la conductibilité ou au moins une électrode ionosélective.

8. Dispositif selon la revendication 7, caractérisé en ce que l'électrode ionosélective est une électrode sélective vis-à-vis du sodium, du potassium, du pH, sensible à $CO_2$, à $HCO_3$ et/ou sélective vis-à-vis du calcium.

9. Dispositif selon la revendication 3, caractérisé en ce que du récipient de concentré (22) part une deuxième canalisation dans laquelle est intercalée une deuxième pompe à concentré, en ce que la première pompe à concentré (24) commandée par une unité de commande fait circuler une première quantité de concentré, qui conduit à une composition inférieure de liquide de dialyse, encore tolérée par le patient, et en ce que la deuxième pompe à concentré est réglée par l'unité de réglage (36) à un signal différentiel de l'unité de différentiation (64).

FIG.1

FIG.2